# EUROPEAN PATENT APPLICATION

(11) **EP 4 406 936 A1**
(43) Date of publication of application: **31.07.2024**
(21) Application number: 22904454.0
(22) Date of filing: 21.10.2022
(51) Int. Cl.: C07C 227/42, C07C 229/34, C12P 13/22

(54) **METHOD FOR PRODUCING TYROSINE FROM FERMENTED BROTH**

(30) Priority: 09.12.2021 KR 20210176117
(71) Applicant: CJ CheilJedang Corporation, Seoul 04560 (KR)
(72) Inventor: KANG, Seung Hoon, Seoul 04560 (KR); PARK, Young Soo, Seoul 04560 (KR); LEE, Chung Yup, Seoul 04560 (KR)
(74) Representative: Regimbeau
(86) International application number: PCT/KR2022/016122
(87) International publication number: WO 2023/106620

(57) **Abstract**

The present invention relates to a method for producing tyrosine with high purity from a fermentation broth containing tyrosine produced by microbial fermentation, wherein the method comprises: obtaining a tyrosine solution by adding a basic solution to a microbial fermentation broth containing tyrosine; obtaining primary tyrosine crystals by neutralization-crystallization by adding an acidic solution to the tyrosine solution; obtaining a tyrosine re-dissolved solution by adding a basic solution to the primary tyrosine crystals; and obtaining secondary tyrosine crystals by neutralization-crystallization by adding an acidic solution to the tyrosine re-dissolved solution.

## Description

### [Technical Field]

The present invention relates to a method for producing tyrosine with high purity from a fermentation broth containing tyrosine produced by microbial fermentation.

### [Background Art]

Tyrosine, an essential amino acid, has the lowest solubility in water among amino acids because it has a polar hydroxyphenyl group, and is an amino acid having a needle-shaped or plate-shaped crystal structure during crystallization in water.

Tyrosine is produced by way of a hydrolysis method, a chemical synthesis method, an enzymatic method, or a microbial fermentation method.

As disclosed in U.S. Patent No. 2650242, it is common to produce tyrosine through decolorization and crystallization after protein hydrolysis in animal hair, *etc.*, and tyrosine can be prepared via chemical synthesis. However, since DL-tyrosine is produced during the manufacturing process, it is not suitable industrially because a complicated separation process is required in order to obtain L-tyrosine.

The enzymatic method uses phenol, pyruvic acid, ammonia or phenol, and L-serine as starting materials to produce tyrosine by the catalytic action of a tyrosine phenol-degrading enzyme, but the technology for controlling the activity of the enzyme during the reaction is insufficient.

The microbial fermentation method requires a method for preparing L-tyrosine through microbial strain fermentation using a carbon source such as glucose or raw sugar as a raw material, or a process for separating and purifying tyrosine from a fermentation broth containing tyrosine.

Chinese Patent Application No. CN 111039808 A discloses a method of extracting tyrosine from a fermentation broth, but since ammonia water is used to dissolve crystals in the fermentation broth, it is difficult to prepare a tyrosine solution in high concentration. Therefore, additional concentration for crystallization is required, and when an acid is slowly added to neutralize and crystallize under basic conditions, which is a general neutralization method, thin, needle-shaped tyrosine is produced, and thus, there is a problem in that a product with high wet crystal moisture and low content (less than 98%) is produced.

### [Disclosure]

### [Technical Problem]

The present inventors conducted a study on a method for efficiently separating and purifying high-purity and high-quality tyrosine from a fermentation broth containing tyrosine, and developed a method for preparing high-purity tyrosine crystals through dissolution of tyrosine using a basic compound.

It is one object of the present invention to provide a method for producing high-purity and high-quality tyrosine from a microbial fermentation broth.

It is another object of the present invention to provide tyrosine crystals produced from a microbial fermentation broth.

### [Technical Solution]

One aspect of the present invention provides a method for producing tyrosine from a microbial fermentation broth, including:
obtaining a tyrosine crystal slurry as a tyrosine solution by adding a basic solution to a microbial fermentation broth containing tyrosine;
obtaining primary tyrosine crystals by neutralization-crystallization by adding an acidic solution to the tyrosine solution;
obtaining a tyrosine re-dissolved solution by adding a basic solution to the primary tyrosine crystals; and
obtaining secondary tyrosine crystals by neutralization-crystallization by adding an acidic solution to the tyrosine re-dissolved solution.

As used herein, the term "microbial fermentation broth" means a microbial culture containing tyrosine produced by a microorganism obtained by culturing a microorganism having tyrosine producing ability, and may include a slurry in which tyrosine separated from the microbial culture is dispersed in water.

As used herein, the term "tyrosine solution" refers to a solution obtained by dissolving tyrosine having low solubility in water in a basic solution such as a strong base. The term "tyrosine re-dissolved solution" means a solution in which tyrosine crystals obtained after primary crystallization are dissolved by adding a strong base.

As used herein, the term "neutralization-crystallization" refers to reacting a substance that is poorly soluble or insoluble in water depending on pH with an acid or base in the presence of water to crystallize the substance. The term "simultaneous neutralization-crystallization" means that a tyrosine solution to be crystallized and an acid are simultaneously added to a vessel such as a crystal tube or a crystallization tank and mixed while maintaining a specific pH to precipitate crystals.

In one embodiment of the present invention, the microbial fermentation broth is obtained by fermentation culture of a microorganism having tyrosine producing ability, and the fermentation culture may be performed by fed-batch culture, batch culture, repetitive fed-batch, or continuous culture. The fermentation medium used can be optimized according to the production strain.

In one embodiment of the present invention, the step of obtaining a tyrosine solution may include adding a basic solution to a pH of 11 to 12. A strong base may be added directly to the microbial fermentation broth containing tyrosine, or a base, for example, 20% to 50%, or 40% NaOH or KOH, is added to a slurry obtained by adding water to tyrosine recovered by high-speed centrifugation of the microbial fermentation broth to obtain a tyrosine solution at pH 11 to 12. Since tyrosine has low solubility in water, it can be dissolved at a high concentration by adding a strong basic solution such as NaOH or KOH.

In one embodiment of the present invention, the step of obtaining primary tyrosine crystals may include adjusting the pH of the mixed solution to within 5 to 7 by mixing while simultaneously adding the tyrosine solution and an acidic solution to a crystallization tank.

In one embodiment of the present invention, the step of obtaining a tyrosine re-dissolved solution may include dissolving the primary tyrosine crystals by adding a basic solution to the primary tyrosine crystals so that the pH of the mixed solution is 11 or higher.

In one embodiment of the present invention, the step of obtaining secondary tyrosine crystals is performing neutralization-crystallization by simultaneously mixing the tyrosine re-dissolved solution and an acidic solution to adjust the pH of the mixed solution to be 5 to 7.

In one embodiment of the present invention, the secondary neutralization-crystallization may be performed at 60°C to 80°C or 70°C to 80°C.

In one embodiment of the present invention, the secondary neutralization-crystallization may be performed at pH 5 to 6, or pH 5.5 by raising the temperature of the tyrosine solution or the tyrosine re-dissolved solution to 60°C to 80°C and adding a dilute acidic solution.

In one embodiment of the present invention, the microbial fermentation broth may be a culture containing 35 g/L or more of tyrosine produced by culturing a strain having tyrosine producing ability, or a slurry obtained by adding water to the tyrosine crystals recovered by high-speed centrifugation of the culture.

In one embodiment of the present invention, high-speed centrifugation for recovering tyrosine from the microbial fermentation broth may be performed at 1,000 g to 5,000 g using a decanter.

In one embodiment of the present invention, high-speed centrifugation for recovering tyrosine from the microbial fermentation broth may be performed at 2,000 g or more.

In one embodiment of the present invention, the method may further include obtaining a filtrate by filtering the tyrosine solution, prior to obtaining the primary tyrosine crystals.

In one embodiment of the present invention, the method may further include a step of subjecting the filtrate to ultrafiltration.

In one embodiment of the present invention, the step of obtaining a filtrate from the tyrosine solution may include filtering through a ceramic filtration membrane having a size of 0.1 microns to remove cellular components from the tyrosine solution, and the method may further include passing the filtrate through an ultrafiltration (UF) membrane D 1000-500,000 to further remove protein components, *etc.* from the filtrate.

In one embodiment of the present invention, the strong basic solution may be NaOH or KOH, and may be used at a concentration of 20% to 50%, or 40%.

In one embodiment of the present invention, the acidic solution may be acetic acid, hydrochloric acid, nitric acid, or sulfuric acid, and may be used in a concentration of 40% or less.

In one embodiment of the present invention, the method may further include decolorizing the tyrosine re-dissolved solution, prior to obtaining secondary tyrosine crystals.

In one embodiment of the present invention, the UV-VIS absorbance of the decolorized tyrosine re-dissolved solution may be 0.0001 to 0.1 at 430 nm, for example, 0.05 or less.

In one embodiment of the present invention, the decolorizing step may include filtering the tyrosine re-dissolved solution to obtain a filtrate; and decolorization by adding activated carbon to the filtrate to obtain a decolorization re-dissolved solution.

In one embodiment of the present invention, the decolorizing step may further include further decolorizing the decolorization re-dissolved solution through a basic anion exchange resin.

In one embodiment of the present invention, in order to decolorize the color of the tyrosine re-dissolved solution, activated carbon may be added to the re-dissolved solution, treated at 60°C for about 1 hour, filtered with a filter press, and further passed through a basic anion resin column.

In one embodiment of the present invention, the method may include performing neutralization-crystallization of the tyrosine re-dissolved solution, and then filtering the obtained reaction solution through a basket separator, and washing tyrosine crystals with water corresponding to 20% of the filtration volume.

In one embodiment of the present invention, the tyrosine produced by the method may be needle-shaped crystals having a crystal size of 70 microns or more and a moisture content of 15% or less.

In one embodiment of the present invention, the method may further include drying the obtained tyrosine crystals to a moisture content of 0.5% or less in a fluidized bed dryer.

Another aspect of the present invention provides tyrosine in needle-shaped crystals having a size of 70 microns or more and a moisture content of 15% or less, obtained by dissolution and simultaneous neutralization-crystallization of tyrosine by a strong base from a microbial fermentation broth.

### [Advantageous Effects]

The method according to one embodiment of the present invention can prepare needle-shaped tyrosine crystals with high purity by dissolving the tyrosine crystals produced in the high-concentration fermentation process with a strong alkali without additional water input, and thus, it is possible to reduce the capacity of the storage tank and the decolorization equipment required during the purification process, and to reduce the energy cost by concentration in the known technology. In addition, the method according to one embodiment of the present invention lowers the wet crystal moisture of the separated crystals in the crystallization process for commercialization, and allows to easily produce high-purity tyrosine crystals (98.5% or more).

### [Brief Description of Drawings]

FIG. 1 shows a flow chart from the tyrosine fermentation broth to the step of obtaining primary tyrosine crystals of the method according to one embodiment of the present invention.
FIG. 2 shows a flow chart from primary tyrosine crystals to the step of obtaining secondary tyrosine crystals of the method according to one embodiment of the present invention.
FIG. 3 shows (a) primary tyrosine crystals obtained according to one embodiment of the present invention and (b) primary tyrosine crystals obtained according to Comparative Example (Chinese Patent Application No. 111039808).
FIG. 4 shows (a) secondary tyrosine crystals obtained according to one embodiment of the present invention and (b) secondary tyrosine crystals obtained according to Comparative Example (Chinese Patent Application No. 111039808).

### [Detailed Description of Preferred Embodiments]

Hereinafter, the present invention will be described in detail by way of Examples. However, these Examples are provided for illustrative purposes only, and the scope of the invention is not intended to be limited to or by these Examples.

### Example 1. Preparation of Tyrosine Crystals from Tyrosine

### Fermentation Broth

In this example, a high-concentration solution of tyrosine was obtained from a fermentation broth containing tyrosine having low water solubility, and crystallization was performed therefrom to prepare tyrosine crystals.

### (1) Solubility of Tyrosine

Since tyrosine has low solubility in water, it was dissolved by adding a base to obtain a high-concentration solution. The dose, pH, and tyrosine solubility of the fermentation broth containing tyrosine were compared using 30% ammonia and 50% NaOH. Table 1 below shows the results.

**[Table 1]**

| | pH | Tyr (g/L) | Dose (mL) | Addition (v/v) | | pH | Tyr (g/L) | Dose (mL) | Addition (v/v) |
|---|---|---|---|---|---|---|---|---|---|
| 30% Ammonia | 5.5 | 0.7 | 0 | 0.0% | 50 % NaO H | 5.5 | 0.7 | 0 | 0.0% |
| | 9.0 | 1.5 | 2.5 | 0.5% | | 9.0 | 4.2 | 3 | 0.6% |
| | 10.0 | 15.2 | 20 | 4.0% | | 10.0 | 66.0 | 17 | 3.4% |
| | 10.3 | 16.3 | 60 | 12.0% | | 11.0 | 69.6 | 22 | 4.4% |
| | 10.5 | 24.3 | 124 | 24.8% | | 12.0 | 70.0 | 25 | 5.0% |

In order to obtain a high-concentration tyrosine solution, a high-concentration tyrosine solution was obtained with a small amount of addition, when 50% NaOH was used rather than 30% ammonia.

### (2) Preparation of Tyrosine Solution

In this example, a fermentation broth containing tyrosine was prepared by culturing *Corynebacterium glutamicum* CM06-0112 (KCCM12708P), a high-concentration, high-productivity tyrosine-producing strain based on *Corynebacterium*, for 60 hours in batch culture in the fermentation process. It was cultured such that tyrosine in the fermentation broth was 85 g/L.

A high-concentration tyrosine solution was prepared by adding a strong basic solution (50% NaOH or 50% KOH) to the tyrosine fermented product obtained through fermentation as confirmed in (1), and a tyrosine solution was obtained by passing through a ceramic membrane in order to remove the remaining cellular components and residual proteins in the solution.

In order to prepare tyrosine crystals of high purity (98.5% or more) from the fermentation broth obtained by fermentation of a strain having tyrosine producing ability, two crystallizations were required.

### (3) Primary Crystallization of Tyrosine Solution

Primary crystallization is a crystallization process for effectively removing other amino acids and ionic components contained in the fermentation broth. In (2), a strong basic solution was added to dissolve tyrosine, and the basic tyrosine solution obtained by removing the cells and a dilute sulfuric acid solution were simultaneously added into a crystallization tank containing purified water, which allows easy mixing, to momentarily adjust the pH to 5 to 7 and thereby obtain tyrosine crystals in the form of granules by simultaneous neutralization-crystallization. The tyrosine crystal formed therefrom is easily precipitated, and has the advantage of showing low wet crystal moisture and high purity compared to the known crystallization process.

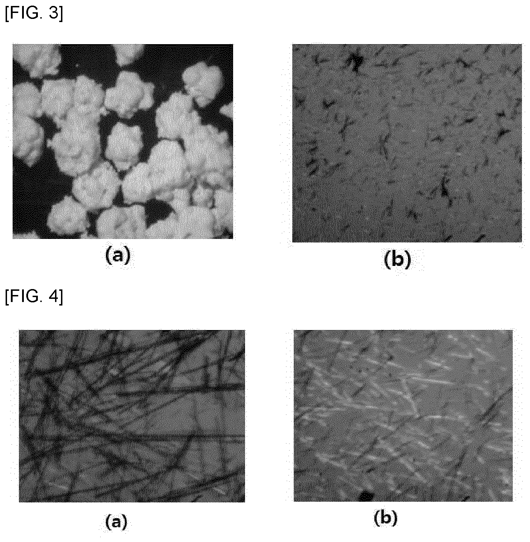

In order to compare the simultaneous neutralization-crystallization and the isoelectric neutralization-crystallization, as described in Chinese Patent Application CN 111039808 A, an acid solution (hydrochloric acid or acetic acid) was slowly added to an alkaline process solution from which cells have been removed to perform isoelectric crystallization at pH 5.7. The tyrosine crystals produced from the simultaneous neutralization-crystallization were in the form of granules, and the crystals were could be easily separated because of their large size, and had high tyrosine content. However, it was confirmed that very small and thin tyrosine crystals were formed from the isoelectric neutralization-crystallization, making it difficult to separate the crystals from the solution, and the content was low. Table 2 below shows the wet crystal moisture, crystal grain size, and content according to the primary crystallization method, and the shape of the crystal is shown in FIGS. 3(a) and 3(b).

**[Table 2]**

| Crystallization Method | Simultaneous Neutralization-Crystallization | Isoelectric Neutralization-Crystallization |
|---|---|---|
| Moisture of Separated Crystals (%) | 28% | 65% |
| Crystal Size (µm) | 690 | 30 |
| Crystal Purity (%) | 95 | 80 |
| Crystal Shape | Granules | Small Needle-Shape |

The crystals separated through the primary crystallization showed a purity level of 95% (based on HPLC) and a crystal recovery rate of 97%.

### (4) Decolorization and Secondary Crystallization of Tyrosine Solution

The primary tyrosine crystals obtained in (3) were re-dissolved in a strong base solution to obtain a tyrosine re-dissolved solution, and decolorization was performed using activated carbon and anionic resin.

Since pure tyrosine crystals are very small, needle-shaped crystals, neutralization-crystallization, which proceeds slowly from basic to neutral, has problems in that it is difficult to dry the crystals due to high wet crystal moisture during crystal separation, and the content is not high. In order to overcome this problem, a crystallization process capable of producing tyrosine with low wet crystal moisture and high purity by increasing the thickness of the crystal while maintaining the tyrosine crystal form was developed. Specifically, in order to increase the thickness of the tyrosine crystals, the temperature of the crystallization tank containing the stirring purified water was heated to 70°C to 80°C, and then the decolorized tyrosine re-dissolved solution and a dilute acid (H₂SO₄, HCl, AcOH) were simultaneously added to momentarily adjust the pH of the tyrosine re-dissolved solution to 5 to 7 and thereby produce needle-shaped tyrosine crystals with increased thickness, which were recovered with a basket separator and dried to prepare high-purity tyrosine for food.

As in (3), in order to compare the simultaneous neutralization-crystallization and the isoelectric neutralization-crystallization, as described in Chinese Patent Application CN 111039808 A, an acid solution (hydrochloric acid or acetic acid) was slowly added to an alkaline process solution from which the cells were removed to perform the isoelectric crystallization at pH 5.7.

The properties of the crystals obtained by simultaneous neutralization-crystallization and isoelectric crystallization of the decolorized decolorizing re-dissolved solution are shown in Table 3, and the shape of the crystals are shown in FIGS. 4(a) and 4(b), respectively.

**[Table 3]**

| Crystallization Method | Simultaneous Neutralization-Crystallization | Isoelectric Neutralization-Crystallization |
|---|---|---|
| Temperature (°C) | 70-80 | 30-60 |
| Moisture of Separated Crystals (%) | 15% | 60% |
| Crystal Size (µm) | 70 | 30 |
| Crystal Content (%) | 98.5 | 97.0 |
| Crystal Shape | Thick Needle-Shape | Thin Needle-Shape |

### Example 2. Dissolution of High-Concentration Tyrosine Crystals in Fermentation Broth

Based on the results of Example 1, dissolution of tyrosine crystals was performed under predetermined conditions.

Specifically, 5,000 mL of tyrosine fermentation broth (80 g/L) was placed in a flask equipped with a thermometer and a stirrer and stirred at 30°C. At this time, the pH of the fermentation broth was 6.5 to 7.0, and when 220 mL of a strong base of 50% NaOH or 50% KOH was added, the color of the fermentation broth changed to dark brown at a pH of 11.0, and all crystals were dissolved (69.6 g/L).

When 1,240 mL of 30% aqueous ammonia, which is a weak base, was added instead of 220 mL of 50% NaOH or 50% KOH, which is a strong base, the pH of the process solution was 10.5. At this time, the solubility of tyrosine was 24.3 g/L, and all tyrosine crystals in the fermentation broth were not dissolved.

The solubility according to the pH of the tyrosine solution is shown in Table 4.

**[Table 4]**

| | | | | | | | | | |
|---|---|---|---|---|---|---|---|---|---|
| pH | 7.0 | 8.0 | 9.0 | 10.0 | 10.4 | 10.5 | 10.6 | 10.8 | 11.0 |
| Concentratio n (g/L) | 0.5 | 0.7 | 1.3 | 5.2 | 8.4 | 24.3 | 27.8 | 52.1 | 69.6 |

In the case of using a weak base for dissolution of tyrosine, an excess amount must be administered, and the solubility of tyrosine crystals relative to the dose is low compared to the case of using a strong base, and thus it is inefficient.

### Example 3. Primary Crystallization by Primary Simultaneous

### Neutralization

As described in Example 2, after heating the fermentation broth, in which the tyrosine crystals were dissolved by adding a strong base, to 55°C, the cells were removed at operating conditions of 2 bar using equipment (TAMI INDUSTRIES) equipped with a 0.12 micron ceramic membrane filter to prepare a tyrosine solution (75 g/L) for carrying out crystallization was prepared.

After adding 1,000 mL of purified water to a 10,000 mL crystal tube equipped with a thermometer, a stirrer, and a pH meter (METTLER TOLEDO), it was stirred at 30°C. Using two metering pumps, 5,000 mL of tyrosine solution and 420 mL of 32% sulfuric acid were continuously introduced into the crystal tube at the same time for 1 or 2 hours so that the pH in the crystal tube was maintained at 5.0 to 7.0.

After completion of the addition of the tyrosine solution and sulfuric acid, the mixture was further stirred for about 30 minutes, and the crystals were separated at 1,500 rpm for 10 minutes using a basket separator (KOKUSAN H-122), and the mother solution attached to the crystal surface was washed with 500 mL of purified water to obtain 560 g of primary tyrosine crystals (yield 93%).

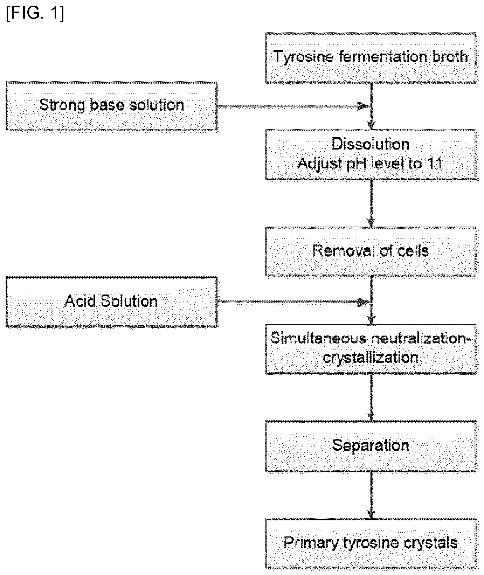

FIG. 1 illustrates a schematic diagram of a method for obtaining primary tyrosine crystals from a tyrosine fermentation broth.

The obtained crystals had a moisture content of 30% based on LOD (Loss on Drying) at 105°C, and a content of 95.8% based on HPLC.

### Example 4. Secondary Crystallization Method by Secondary Simultaneous Neutralization

4,000 mL of purified water was added to 560 g of the primary tyrosine crystals obtained in Example 3, and heated to 55°C. 50% NaOH was added thereto to completely dissolve the crystals at pH 11.0-11.5 to obtain a tyrosine re-dissolved solution. In order to remove the color and protein component of the secondary crystallization solution, 0.4% of activated carbon was added to the re-dissolved solution, stirred for 1 hour, and filtered using Whatman GLASS MICROFIBER FILTERS (GF/F). The obtained filtrate was passed through a strong basic anion resin column (TRILITE AMP26) once again to prepare a tyrosine re-dissolved solution for secondary crystallization. The tyrosine concentration of the tyrosine re-dissolved solution for crystallization, the obtained crystallization feed, was 35 g/L, and the absorbance measured at 430 nm with a UV-VIS spectrophotometer was 0.02.

After adding 1,000 mL of purified water to a 10,000 mL crystal tube equipped with a thermometer, a stirrer, and a pH meter, it was stirred at 75°C. Using two metering pumps, 5,000 mL of the above-prepared crystallization feed and 480 mL of 32% sulfuric acid were continuously added for 3 hours so that the pH in the crystal tube was maintained at 6.0. After completion of the addition of the crystallization feed, the temperature of the feed was lowered to 55°C and the feed was separated at 1,500 rpm for 10 minutes using a basket separator (KOKUSAN H-122) to obtain 380 g of secondary tyrosine crystals (yield 91%).

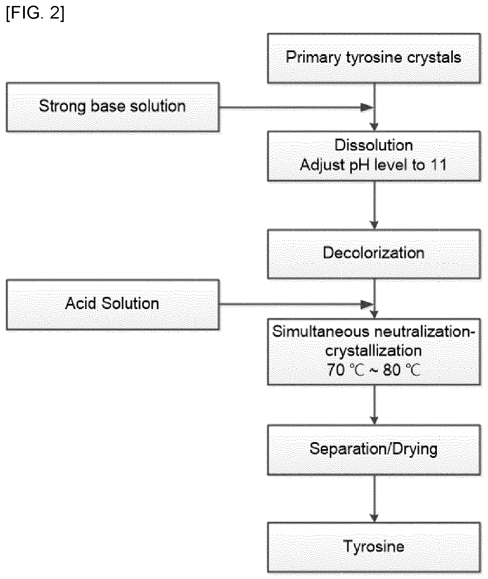

FIG. 2 illustrates a schematic diagram of a method for obtaining secondary tyrosine crystals.

The obtained crystals had a moisture content of 10% based on LOD at 105°C and a content of 99.0% based on HPLC.

## Claims

1. A method for producing tyrosine, comprising:
obtaining a tyrosine solution by adding a basic solution to a microbial fermentation broth containing tyrosine;
obtaining primary tyrosine crystals neutralization-crystallization by adding an acidic solution to the tyrosine solution;
obtaining a tyrosine re-dissolved solution by adding a basic solution to the primary tyrosine crystals; and
obtaining secondary tyrosine crystals by neutralization-crystallization by adding an acidic solution to the tyrosine re-dissolved solution.

2. The method of claim 1, wherein the step of obtaining the tyrosine solution comprises adding a basic solution such that the pH of the tyrosine solution is equal to or higher than 11.

3. The method of claim 1, wherein the step of obtaining primary tyrosine crystals comprises adjusting the pH of the mixed solution to within 5 to 7 by mixing while simultaneously adding the tyrosine solution and an acidic solution to a crystallization tank.

4. The method of claim 1, wherein the step of obtaining the tyrosine re-dissolved solution comprises dissolving the primary tyrosine crystals by adding a basic solution to the primary tyrosine crystals so that the pH of the mixed solution is 11 or higher.

5. The method of claim 1, wherein the step of obtaining secondary tyrosine crystals is performing neutralization-crystallization by simultaneously mixing the tyrosine re-dissolved solution and an acidic solution to adjust the pH of the mixed solution to be 5 to 7.

6. The method of claim 5, wherein the neutralization-crystallization is performed at 70°C to 80°C.

7. The method of claim 1, further comprising a step of obtaining a filtrate by filtering the tyrosine solution, prior to obtaining the primary tyrosine crystals.

8. The method of claim 7, further comprising a step of ultrafiltration of the filtrate.

9. The method of claim 1, wherein the basic solution comprises at least one selected from the group consisting of NaOH or KOH.

10. The method of claim 1, wherein the acidic solution comprises at least one selected from the group consisting of acetic acid, hydrochloric acid, nitric acid, and sulfuric acid.

11. The method of claim 1, further comprising a step of decolorizing the tyrosine re-dissolved solution prior to obtaining the secondary tyrosine crystals.

12. The method of claim 10, wherein the decolorizing step comprises: filtering the tyrosine re-dissolved solution to obtain a filtrate; and decolorization by adding activated carbon to the filtrate to obtain a decolorization re-dissolved solution.

13. The method of claim 12, wherein the decolorizing step further comprises further decolorizing the decolorization re-dissolved solution through a basic anion exchange resin.

14. The method of claim 1, wherein the tyrosine produced by the method for producing tyrosine is needle-shaped crystals having a crystal size of 70 microns or more and a moisture content of 15% or less.
